# EUROPÄISCHE PATENTSCHRIFT

(11) **EP 0 714 634 B1**
(45) Veröffentlichungstag und Bekanntmachung des Hinweises auf die Patenterteilung: **11.04.2001**
(21) Anmeldenummer: 94810678.6
(22) Anmeldetag: 30.11.1994
(51) Int. Cl.: A61B 17/16

(54) **Anordnung zum Erzeugen von Knochenspänen**
Arrangement for producing bone chips
Arrangement pour la production de copeaux d'os

(43) Veröffentlichungstag der Anmeldung: 05.06.1996
(73) Patentinhaber: Sulzer Orthopädie AG, 6340 Baar (CH)
(72) Erfinder: Grunder, Beat, CH-3078 Richigen (CH)
(74) Vertreter: Sulzer Management AG

(56) Entgegenhaltungen:
- DE-A- 3 808 409
- FR-A- 2 371 185
- US-A- 3 856 219
- US-A- 5 295 992
- DATABASE WPI Week 9035, Derwent Publications Ltd., London, GB; AN 90-266567 KUIB MED INST 'Bone shaving making device' & SU-A-1 521 464 15. November 1989

## Beschreibung

Die Erfindung handelt von einer Anordnung zum Erzeugen von Knochenspänen aus Knochenstücken.

Beim Einsetzen von Knochenimplantaten oder bei Knochenplastiken kommt es vor, dass autologe oder homologe Knochenstücke zu Knochenspänen zerkleinert werden, um damit fehlende Knochen zu ersetzen. Beim Ersatz eines Hüftgelenks kann beispielsweise der Femurkopf nach dem Entfernen von Knorpel und von knochenfremden Material zu Spänen verarbeitet werden, welche dem Hinterfüttern einer künstlichen im Acetabulum eingesetzten Hüftgelenkschale dienen.

So führt die Firma PROTEK AG, CH-3110 Münsingen, eine Knochenmühle in ihrem Programm (Produkteinformation Nr.04/89), die mit zwei liegenden und ineinandergreifenden, als Fräser ausgeführten Zahnwalzen Knochenstücke zerhackt, die durch Verwendung von Sieben das Assortieren der gewünschten Grösse der Fragmente gestattet. Die Zahnwalzen drehen aus Sicherheitsgründen nur, wenn der Späneraum unter den Walzen und das Einfüllrohr über den Walzenfräsern mechanisch verriegelt ist. Diese Art der Späneerzeugung bedingt eine schwere Konstruktion, die nicht so leicht zu reinigen und zu sterilisieren ist. Ultraschallreinigung und Heissluftsterilisation werden ausgeschlossen.

In der Patentschrift US-A-5,295,992 ist ein Bearbeitungswerkzeug beschrieben, mit welchem die Rückseite einer Patella-Scheibe zurückgeschnitten wird. Ein scheibenförmiges Werkzeug mit vorstehenden Schneiden und Durchbrüchen für die Späneabfuhr wird mit einem Bajonettverschluss auf einen rotierenden Zylinder aufgesetzt, der seinerseits über einen angeformten Schaft in das Bohrfutter einer Handbohrmaschine einsetzbar ist. Ziel der Einrichtung ist es, eine plane Fläche auf der Patella zu erzeugen und Knorpel und Knochenspäne im rotierenden Zylinder zu sammlen. Voraussetzung für eine wirksame Bearbeitung ist, dass die Patella in einer Halteeinrichtung fixiert ist. Das Dokument gibt keine Auskunft, wie lose Knochenstücke zu Knochenspänen verarbeitet werden können.

Aufgabe der Erfindung ist es, eine Anordnung zum Erzeugen von Knochenspänen aus Knochenstücken zu schaffen, die aus wenigen Einzelteilen besteht und leicht zu reinigen und zu sterilisieren ist.

Diese Aufgabe wird mit den Merkmalen des unabhängigen Anspruchs 1 gelöst.

Ein Vorteil dieser Anordnung ist, dass ein Fräswerkzeug, welches auf seiner Rückseite nicht verletzt, mit seiner Vorderseite in einen geschlossenen Einfüllraum hineinbewegt wird, während sich auf seiner Rückseite gleichmässig geschnittene Knochenspäne sammeln. Es ist ein weiterer Vorteil, dass die Schneiden in einer Scheibe versenkt sind, was das Unfallrisiko soweit herabsetzt, dass die Frässcheibe in einer Handbohrmaschine eingesetzt werden kann, um sie an den Einfüllraum zu bringen.

Durch die Verwendung einer Rutschkupplung, welche in den Antriebszapfen integriert ist oder welche bei Ueberbelastung am Gehäuse dessen Mitdrehen gestattet, wird das Unfallrisiko weiter herabgesetzt. Dadurch kann ein im Operationsraum verfügbarer und von Hand gehaltener Bohrantrieb verwendet werden, während die Vorrichtungsteile einfache und leicht zugängliche Formen aufweisen.

Weitere vorteilhafte Ausbildungen der Erfindung sind in den abhängigen Ansprüchen 2 bis 10 aufgeführt. So erweist es sich als Vorteil, wenn die Frässcheibe im Zentrum und/oder am Rand in Vorschubrichtung vorsteht oder wenn der Anpresskörper ausserhalb des Bereichs der Schneiden vorsteht, um den Anpresskörper ohne Kontakt vor den Schneiden zurückzuschieben während die Schneiden den Einfüllraum schneidend überstreichen und aus den zwischen Gehäuse, Anpresskörper und Frässcheibe gehaltenen Knochenstücken Späne herausschneiden, deren Querschnitt durch die Abmessungen der vorstehenden Schneiden bestimmt ist. Durch auswechselbare Frässcheiben mit unterschiedlichen Schneidenabmessungen können gezielt bestimmte Spanquerschnitte und -Formen hergestellt werden. Die Späne sammeln sich in einem Späneraum hinter der Frässcheibe und werden mit dem Herausziehen der Frässcheibe ausgebracht. Das Gehäuse hat auf der Innenseite die Form eines Kreiszylinders mit abgesetztem Boden und führt die Frässcheibe und den Anpresskörper in der Vorschubrichtung, die mit der Einfüllrichtung und Zylinderachse parallel ist. Dadurch, dass der Anpresskörper sich über das Zentrum der Fräserscheibe erstreckt, findet kein Quetschen im Zentrum statt und jede der zueinander versetzten und sich in der Laufbahn überdeckenden Schneiden hat eine endliche Schnittgeschwindigkeit. Gleichzeitig ist es damit möglich, im Zentrum der Frässcheibe einen Stütz- und Zentrierstift vorzusehen, der sich auf dem Anpresskörper abstützt und diesen in Vorschubrichtung vor sich her schiebt. Dabei leistet der Anpresskörper Widerstand, indem er durch ein Federelement oder durch eine Reibungsbremse zum Gehäuse abgestützt ist. Dadurch, dass der Gehäuseboden abnehmbar gehalten ist, sind alle Flächen zur Reinigung und Kontrolle gut einsehbar und wenige, in sich stabile Einzelteile vorhanden, die leicht zu sterilisieren sind. Die vertikale Aufstellung ermöglicht es durch blosses Zusammenstecken und eventuelles Verriegeln den betriebsbereiten Endzustand zu erreichen. Einzig der Fräser muss in einem Spannfutter befestigt werden. Der Fräser wird von Hand in seine Arbeitsstellung gebracht, wobei sich die Teile ohne zusätzliche Lager und Rückführungen gegenseitig direkt stützen und durch die Verwendung einer Rutschkupplung vor Ueberbelastung geschützt sind. Diese Betriebsart erfolgt ohne merkbaren Verschleiss, da die pro Patient notwendige Spänemenge in der Regel in einem Bruchteil von einer Minute geschnitten ist. Die Späne selbst fallen gut reproduzierbar als Bröckelspäne oder Scherspäne mittlerer Länge an.

Im folgenden wird die Erfindung anhand von Ausführungsbeispielen beschrieben. Es zeigen:
- Fig.1: schematisch einen erfindungsgemässen Fräser mit einer Frässcheibe, die Schneiden in Durchbrüchen aufweist;
- Fig.2: schematisch ein erfindungsgemässes Gehäuse mit Einfüllraum, Anpresskörper und Federelement;
- Fig.3: schematisch eine Ansicht von unten auf eine Frässcheibe gemäss Fig.1;
- Fig.4: schematisch einen Ausschnitt aus einer Randpartie einer Frässcheibe gemäss Fig.1;
- Fig.5: schematisch einen Schnitt durch eine Schneide in Fig.4, bei dem Knochenstück und Knochenspan ergänzt sind;
- Fig.6: schematisch eine Draufsicht auf einen Einfüllraum und Anpresskörper gemäss Fig.2, und
- Fig.7: schematisch eine Reibungsbremse zwischen Anpresskörper und Gehäuse.
- Fig.8: schematisch ein Fräswerkzeug mit integrierter Rutschkupplung.
- Fig.9: schematisch einen Querschnitt durch einen Anpresskörper, der die Seitenwände zu einem Einfüllraum für Knochenstücke bildet.

In den Figuren ist ein Scheibenfräser gezeigt, der in einer Scheibe eingelagerte Schneiden und Durchbrüche aufweist, um aus Knochenstücken Späne zu schneiden. Die Knochenstücke werden in einer Einfüllrichtung in einem als Sackloch ausgeführten Einfüllraum eingelegt, der durch den Gehäuseboden und seitlich durch eine zylindrische Gehäusewand und/oder einen Anpresskörper begrenzt ist. Der in einer Handbohrmaschine einsetzbare Scheibenfräser stützt sich an einer Zylinderfläche im Gehäuse ab und überdeckt Anpresskörper und Einfüllraum. Beim Anpressen des Scheibenfräsers in Vorschubrichtung schiebt dieser den Anpresskörper entsprechend der Abnahme der Knochenstücke vor sich her und sammelt auf seiner Rückseite die frisch geschnittenen Knochenspäne in einem Späneraum.

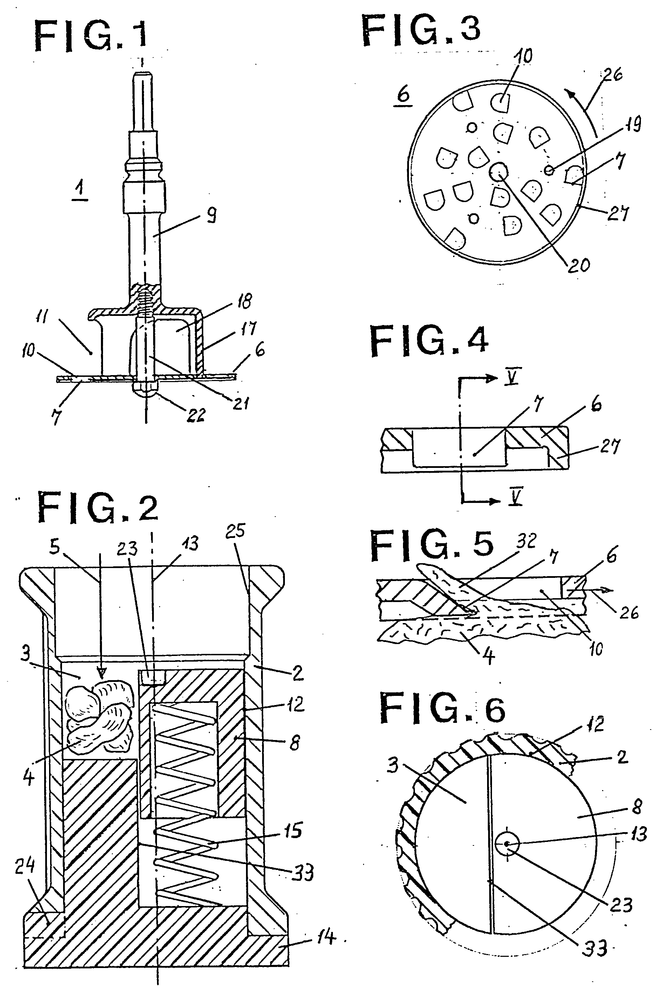

In Fig.1 ist ein Fräser 1 mit einem Antriebszapfen 9 versehen, der in das Spannfutter einer Handbohrmaschine passt, die nicht gezeigt ist. Der Antriebszapfen 9 erweitert sich zu einer Glocke 17 mit Aussparungen 18 und Stegen, die in Zapfen enden, welche in die Aufnahmebohrungen 19 in Fig.3 einer Frässcheibe 6 passen, um ein Drehmoment in Drehrichtung 26 zu übertragen. Die Frässcheibe 6 ist durch eine Befestigungsschraube 21 über ein Loch 20 mit dem Antriebszapfen 9 auswechselbar verankert. Die Frässcheibe 6 hat Schneiden 7 mit zugehörigen Durchbrüchen 10 eingelagert, wobei die Schneiden 7 so verteilt sind, dass sich ihre Laufflächen überdecken. In Figur 4 ist eine Frässcheibe 6 mit einem vorstehenden Rand 27 gezeigt, der weiter als die Schneiden 7 in Vorschubrichtung vorsteht. Innerhalb und ausserhalb der Glocke 17 besteht ein Späneraum 11, um frisch geschnittene Knochenspäne 32 zu sammeln.

Figur 2 zeigt ein Gehäuse 2, das innen als Kreiszylinder 12 ausgeführt ist. Ein abnehmbarer Boden 14 ist über einen Vorsprung 24 drehfest steckbar mit dem Gehäuse 2 verriegelt. Die Kreisfläche vom Boden 14 ist innen im Gehäuse 2 abgesetzt, um einerseits den Boden für einen Einfüllraum 3 für Knochenstücke 4 zu bilden und andererseits mit einem Absatz eine Führungsfläche 33 für einen Anpresskörper 8 zu bilden. Der Anpresskörper 8 ist zwischen der Führungsfläche 33 und der inneren Mantelfläche vom Kreiszylinder 12 drehfest und in der Richtung der Zylinderachse 13 verschiebbar geführt. Er begrenzt mit seiner Seitenfläche den Einfüllraum 3 und wird durch ein Federelement 15 in einer Anfangslage gehalten. Der Einfüllraum 3 nimmt weniger als die Hälfte der Kreisfläche vom Zylinder 12 ein, damit im Zentrum auf dem Anpresskörper 8 eine Oeffnung 23 zum Zentrieren des Fräsers 1 geschaffen wird, die den Fräser an dem verrundeten Kopf 22 der Befestigungsschraube 21 seitlich führen hilft. Der Fräser 1 wird in einer Handbohrmaschine eingespannt.Ein über die Schneiden 7 vorstehender Rand 27 verhindert, dass die Schneiden in den Anpresskörper 8 hineinfahren. Da gleichzeitig mit dem Rand 27 der Kopf 22 in der Oeffnung 23 des Anpresskörpers aufsitzt, wird letzterer vor der Fräserscheibe 6 hergestossen, während der Anpresskörper 8 den Einfüllraum 3 seitlich entsprechend dem Vorschub begrenzt.

Die in den Einfüllraum 3 eingefüllten Knochenstücke 4 bestimmen entsprechend dem Abtrag der Knochenspäne 32, wie schnell der Fräser 1 in Vorschubrichtung, d.h. in der Richtung der Einfüllrichtung 5 gestossen werden kann. Bei einem Gehäuse 2, das sich an seiner Aussenfläche noch mit einer Hand halten lässt, beträgt eine typische Schnittzeit für die Knochenspäne 32 zum Beispiel 15 Sekunden. Mit dem Verringern der Knochenstücke 4 im Einfüllraum 3 bewegt sich der Anpresskörper 8 entgegen der Kraft vom Federelement 15 nach unten. Dünne und flache Knochenstücke 4 benötigen einen höheren Anpressdruck. Eine progressive Anpresskraft wie bei einer Federcharakteristik ist somit erwünscht.

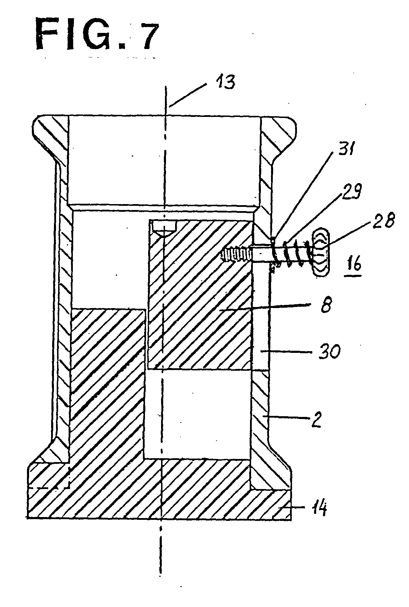

Figur 7 zeigt eine Variante mit einer Reibungsbremse 16 zwischen Anpresskörper 8 und Gehäuse 2. Durch einen zur Achse 13 parallelen Schlitz 30 im Gehäuse 2 ist eine Rändelschraube 28 im Anpresskörper 8 eingeschraubt, die über eine Druckfeder 29 die Gehäusewand zwischen einer Unterlagsscheibe 31 und dem Anpresskörper 8 verpresst. Das Absenken des Anpresskörpers 8 kann somit über die Reibung und zusätzlich von Hand kontrolliert werden. Gleichzeitig dient die Rändelschraube dem Zurückstellen in die Anfangsstellung. Demgegenüber hat eine Ausführung nach Fig.2 den Vorteil, dass sie mit einem Minimum an steckbaren und leicht zu reinigenden Teilen auskommt. Die entstehenden Späne sind Bröckelspäne oder Scherspäne und hängen daher nicht in ihrer Länge vom Abstand der Schneiden 7 auf einer Laufbahn ab. Dadurch, dass der Einfüllraum 3 vor dem Zentrum der Fräserscheibe 6 endet, werden alle Späne mit ausreichender Schnittgeschwindigkeit geschnitten. Die Art der Späne lässt sich durch die Schneidengeometrie resp. durch auswechselbare Fräserscheiben 6 bestimmen. Die fertig geschnittenen Späne sammeln sich im Späneraum 11 und lassen sich mit dem Fräswerkzeug 1 herausheben oder bei schräggestelltem Gehäuse 2 in eine bereitgestellte Schale schütten.

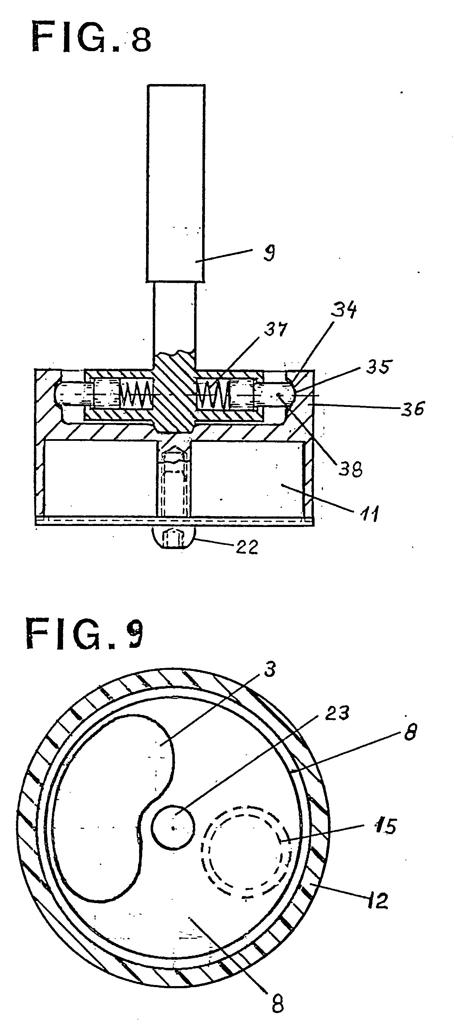

Figur 8 zeigt ein Fräswerkzeug 1, in das eine Rutschkupplung 34 integriert ist, die Vertiefungen 35 in einem Aussenkranz 36 und radial unter Federkraft 37 angepresste Formkörper 38 aufweist, welche bei zu grossem Drehmoment zurückfedern und ein Durchdrehen ermöglichen.

In Figur 9 ist ein Anpresskörper 8 gezeigt, der den Einfüllraum 3 seitlich vollständig umgibt und verhindert, dass Knochensplitter in die Führungsfläche zwischen Kreiszylinder 12 und Anpresskörper 8 gelangen.

## Patentansprüche

1. Anordnung zum Erzeugen von Knochenspänen (32) aus Knochenstücken (4) mit einem Fräswerkzeug (1) und mit einem Gehäuse (2), das einen Einfüllraum (3) für die Knochenstücke (4) aufweist, wobei der Einfüllraum (3) als ein in einer Einfüllrichtung (5) zugängliches Sackloch ausgebildet ist und das Fräswerkzeug (1) eine Scheibe (6) mit Schneiden (7) und Durchbrüchen (10) umfasst, und wobei die Scheibe nach dem Einfüllen der Knochenstücke (4) mit ihrer mit Schneiden besetzten Seite den Einfüllraum (3) und einen den Einfüllraum (3) seitlich begrenzenden und in Einfüllrichtung (5) beweglichen Anpresskörper (8) abdeckt und in Einfüllrichtung (5) beweglich ist.

2. Anordnung nach Anspruch 1, dadurch gekennzeichnet, dass das Fräswerkzeug (1) bei einer Vorschubbewegung in Einfüllrichtung (5) den Anpresskörper (8) vor sich her schiebt, wobei die Frässcheibe im Zentrum und/oder am Rand vorsteht oder wobei der Anpresskörper (8) ausserhalb des Bereiches der Schneiden (7) vorsteht, um den Kontakt zu den Schneiden zu vermeiden.

3. Anordnung nach Anspruch 1 oder 2, dadurch gekennzeichnet, dass die Scheibe (6) so an einem Antriebszapfen befestigt ist, dass für die Späne (32) nach dem Durchlaufen der Durchbrüche (10) ein Späneraum (11) vorhanden ist.

4. Anordnung nach Anspruch 3, dadurch gekennzeichnet, dass das Fräswerkzeug (1) mit seinem Antriebszapfen (9) so ausgebildet ist, dass es in ein Aufspannfutter einer Handbohrmaschine einsetzbar ist.

5. Anordnung nach einem der Ansprüche 1 bis 4, dadurch gekennzeichnet, dass das Gehäuse (2) auf der Innenseite als Kreiszylinder (12) mit Achse (13) in Einfüllrichtung (5) ausgeführt ist, welcher die Fräserscheibe (6) und den Anpresskörper (8) in Einfüllrichtung (5) führt und welcher den Einfüllraum mit einer abgesetzten Bodenfläche begrenzt.

6. Anordnung nach Anspruch 5, dadurch gekennzeichnet, dass der Anpresskörper (8) eine seitliche Begrenzung für einen Einfüllraum (3) bildet, der ausserhalb der Achse (13) des Kreiszylinders (12) liegt.

7. Anordnung nach Anspruch 5, dadurch gekennzeichnet, dass der Zylinder (12) durch einen abnehmbaren Boden (14) abgeschlossen ist.

8. Anordnung nach einem der Ansprüche 1 bis 7, dadurch gekennzeichnet, dass der Anpresskörper (8) durch ein Federelement (15) entgegen der Einfüllrichtung (5) beaufschlagt ist.

9. Anordnung nach einem der Ansprüche 1 bis 7, dadurch gekennzeichnet, dass der Anpresskörper (8) mit einer Reibungsbremse (16) relativ zum Gehäuse (2) gehalten ist.

10. Anordnung nach Anspruch 7, dadurch gekennzeichnet, dass die Elemente Gehäuse (2), Gehäuseboden (14) und Anpresskörper (8) in Einfüllrichtung steckbar verbindbar sind.

## Claims

1. Arrangement for producing bone chips (32) from bone fragments (4) comprising a milling tool (1) and a housing (2) having a filling space (3) for bone fragments (4), characterised in that the filling space (3) is formed as a blind bore accessible in a direction of filling (5), and in that the milling tool (1) comprises a disk (6) with cutters (7) and apertures (10), wherein the disk, after the filling with the bone fragments (4), is movable in the direction of filling (5) and its side occupied by cutters covers over the filling space (3) and a pressing member (8) which laterally bounds the filling space (3) and is movable in the direction of filling (5).

2. Arrangement in accordance with claim 1, characterised in that, upon a movement in the direction of filling (5), the milling tool (1) pushes the pressing member (8) ahead of it, with the milling disc protruding at the centre and/or at the edge or with the pressing member (8) protruding outside the region of the cutters (7) to avoid contact with the cutters.

3. Arrangement in accordance with claim 1 or 2, characterised in that the disk (6) is secured to a drive spigot so that a chip space (11) is present for the chips (32) which have passed through the apertures (10).

4. Arrangement in accordance with claim 3, characterised in that the drive spigot (9) of the milling tool (1) is formed so that it can be fitted into the mounting chuck of a hand drill.

5. Arrangement in accordance with one of the claims 1 to 4, characterised in that the housing (2) is formed on the inside as a circular cylinder (12) with axis (13) in the direction of filling (5), with the cylinder guiding the milling disk (6) and the pressing member (8) in the direction of filling (5) and bounding the filling space with an offset base area.

6. Arrangement in accordance with claim 5, characterised in that the pressing member (8) forms a lateral boundary for a filling space (3) lying outside the axis (13) of the circular cylinder (12).

7. Arrangement in accordance with claim 5, characterised in that the cylinder (12) is closed off by a removable base (14).

8. Arrangement in accordance with one of the claims 1 to 7, characterised in that the pressing member (8) is loaded opposite the direction of filling (5) by a spring element (15).

9. Arrangement in accordance with one of the claims 1 to 7, characterised in that the pressing member (8) is held relative to the housing (2) by a friction brake (16).

10. Arrangement in accordance with claim 7, characterised in that the elements: housing (2), housing base (14) and pressing member (8) are pluggably connectable in the direction of filling.

## Revendications

1. Agencement pour la production de copeaux d'os (32) à partir de fragments osseux (4) avec un outil à fraiser (1) et avec un boîtier (2) qui possède un logement de remplissage (3) pour les fragments osseux (4), où le logement de remplissage est réalisé comme trou borgne accessible dans une direction de remplissage (5) et l'outil à fraiser (1) comprend un disque (6) avec des tranchants (7) et des ajours (10), et où le disque, après le remplissage des fragments osseux (4), par son côté muni de tranchants, couvre le logement de remplissage (3) et un corps d'application (8) délimitant le logement de remplissage (3) latéralement et mobile dans la direction de remplissage (5) et est déplaçable dans la direction de remplissage (5).

2. Agencement selon la revendication 1, caractérisé en ce que l'outil à fraiser (1), lors d'un mouvement de poussée vers l'avant dans la direction de remplissage (5), pousse le corps d'application (8) devant lui, où le disque de fraisage fait saillie au centre et/ou au bord, et où le corps d'application (8) fait saillie à l'extérieur de la zone des tranchants (7) pour éviter le contact avec les tranchants.

3. Agencement selon la revendication 1 ou 2, caractérisé en ce que le disque (6) est fixé de façon à un tenon d'entraînement qu'il existe pour les copeaux (32) après le passage à travers les ajours (10) un logement (11) de copeaux.

4. Agencement selon la revendication 3, caractérisé en ce que l'outil de fraisage (1) avec son tenon d'entraînement (9) est réalisé de façon qu'il peut être placé dans un mandrin de serrage d'une perceuse à main.

5. Agencement selon l'une des revendications 1 à 4, caractérisé en ce que le boîtier (2), au côté intérieur, est réalisé comme cylindre circulaire (12) avec l'axe (13) dans la direction de remplissage (5), qui guide le disque de fraisage (6) et le corps d'application (8) dans la direction de remplissage (5) et qui délimite le logement de remplissage avec une face de fond étagée.

6. Agencement selon la revendication 5, caractérisé en ce que le corps d'application (8) forme une délimitation latérale pour un logement de remplissage (3) qui se situe à l'extérieur de l'axe (13) du cylindre circulaire (12).

7. Agencement selon la revendication 5, caractérisé en ce que le cylindre (12) est fermé par un fond amovible (14).

8. Agencement selon l'une des revendications 1 à 7, caractérisé en ce que le corps d'application (8) est sollicité par un élément à ressort (15) contre la direction de remplissage (5).

9. Agencement selon l'une des revendications 1 à 7, caractérisé en ce que le corps d'application (8) est retenu par un frein à friction (16) relativement au boîtier (2).

10. Agencement selon la revendication 7, caractérisé en ce que les éléments boîtier (2), fond de boîtier (14) et corps d'application (8) peuvent être reliés dans la direction de remplissage par emboîtement.
